# EUROPEAN PATENT APPLICATION

(11) **EP 2 752 155 A1**
(43) Date of publication of application: **09.07.2014**
(21) Application number: 14150278.1
(22) Date of filing: 07.01.2014
(51) Int. Cl.: A61B 5/024, A61B 5/00

(54) **Living body information detection apparatus and living body information detection program**

(30) Priority: 07.01.2013 JP 2013000707
(71) Applicant: Seiko Instruments Inc., Chiba-shi, Chiba (JP)
(72) Inventor: Mugishima, Katsuya, Chiba-shi,, Chiba (JP); Takakura, Akira, Chiba-shi, Chiba (JP); Tsubata, Keisuke, Chiba-shi, Chiba (JP); Koyama, Kazuhiro, Chiba-shi, Chiba (JP); Hasegawa, Takanori, Chiba-shi, Chiba (JP)
(74) Representative: Miller Sturt Kenyon

(57) **Abstract**

To provide a living body information detection apparatus and a living body information detection program capable of allowing a user to easily recognize information based on acquired living body information. A living body information detection apparatus includes an acquisition unit acquiring living body information obtained by detecting a living body signal and a control unit 240 moving a display position of an image associated with the living body information on a display unit based on the magnitude of a value of the living body information obtained by the acquisition unit.

## Description

### BACKGROUND OF THE INVENTION

### FIELD OF THE INVENTION

The present invention relates to a living body information detection apparatus and a living body information detection program.

### BACKGROUND ART

There exists a living body information detection apparatus acquiring living body information of a user during exercise. The living body information detection apparatus is used by, for example, being attached to a user's arm. The living body information detection apparatus displays acquired information on a display unit. The user checks an exercise state of himself/herself by watching information displayed on the display unit.

For example, the living body information detection apparatus measures load data indicating the hardness of exercise content and exercise amount data indicating an amount of exercise during exercise, displaying the exercise state on the display unit based on the measured data. The living body information detection apparatus includes a heart rate sensor detecting pulsation, an attachment device acquiring the detected pulsation at fixed intervals and outputting the pulsation to a management device and the management device displaying data outputted from the attachment device on the display unit. The attachment device is attached to the user's arm.

The management device arranges acquired data in a radial pattern at respective time points when the data was detected. The management device also displays acquired data by circles at different positions according to time points. Then, it is proposed that the management device performs display by determining the size of the circles to be displayed on the display unit so as to indicate the amount of exercise and by determining the color of the circles so as to indicate the hardness of the exercise content grasped for one minute just before the display (for example, refer to Japanese Patent No. 4840147 (Patent Document 1)).

However, it is necessary that the user recognizes the exercise amount for himself/herself from the difference of size or the difference of color of the circles which indicate data in the technique described in Patent Document 1. Accordingly, in the technique described in Patent Document 1, there is a problem that it is difficult to grasp the exercise state of himself/herself intuitively and immediately from the information displayed on the display unit when the user watches the display of the management device while exercising.

### SUMMARY OF THE INVENTION

It is an aspect of the present application to provide a living body information detection apparatus and a living body information detection program capable of allowing the user to easily recognize information based on acquired living body information.

A living body information detection apparatus according to an embodiment of the present application includes an acquisition unit acquiring living body information obtained by detecting a living body signal and a control unit moving a display position of an image associated with the living body information on a display unit based on the magnitude of a value of the living body information obtained by the acquisition unit.

In the living body information detection apparatus according to the embodiment of the present application, the control unit may move the image associated with the living body information on the display unit in at least one direction of a vertical direction, a horizontal direction and an oblique direction with respect to the display unit.

Also in the living body information detection apparatus according to the embodiment of the present application, the control unit may display part of the image associated with the living body information when the image based on the living body information is moved and displayed on the display unit.

Also in the living body information detection apparatus according to the embodiment of the present application, the image associated with the living body information may have a shape which is asymmetrical in the vertical direction.

Also in the living body information detection apparatus according to the embodiment of the present application, the control unit may output a tone corresponding to a position where the image associated with the living body information is moved on the display unit, from a notification unit.

Also in the living body information detection apparatus according to the embodiment of the present application, the living body information may be information indicating the heartbeat, and the control unit may move the display position of the image associated with the heartbeat on the display unit in accordance with the magnitude of the heartbeat.

A living body information detection program allows a computer of a living body information detection apparatus to execute a step of moving a display position of an image associated with living body information on a display unit based on the magnitude of a value of the living body information acquired by an acquisition unit which acquires the living body information obtained by detecting a living body signal.

According to the embodiment of the present application, it is possible to allow the user to easily recognize information based on the acquired living body information.

### BRIEF DESCRIPTION OF THE DRAWINGS

Embodiments of the present invention will now be described by way of further example only and with reference to the accompanying drawings, in which:
Fig. 1 is a block diagram showing a configuration of a heart rate measurement system according to an embodiment;
Fig. 2 is a view showing an example of an outer appearance of a heart rate measurement device;
Fig. 3 is a view showing an example of an outer appearance of an output device;
Fig. 4 is a view for explaining an image displayed on a display unit according to the embodiment;
Figs. 5A to 5D are views for explaining a mark according to the embodiment;
Fig. 6 is a flowchart for explaining an operation example of the heart rate measurement device according to the embodiment; and
Fig. 7 shows examples of images to be displayed on the display unit in accordance with the heart rate, according to the embodiment.

### DESCRIPTION OF THE PREFERRED EMBODIMENTS

Hereinafter, an embodiment of the present invention will be explained with reference to the drawings. In the following embodiment, the explanation will be made by citing a heart rate measurement system as an example of a living body information detection apparatus.

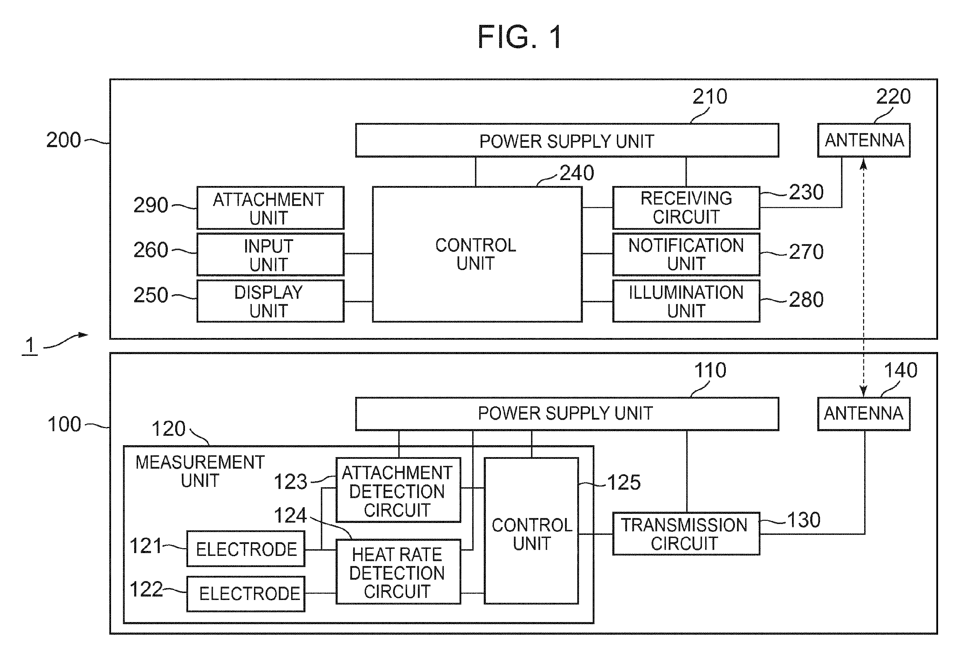

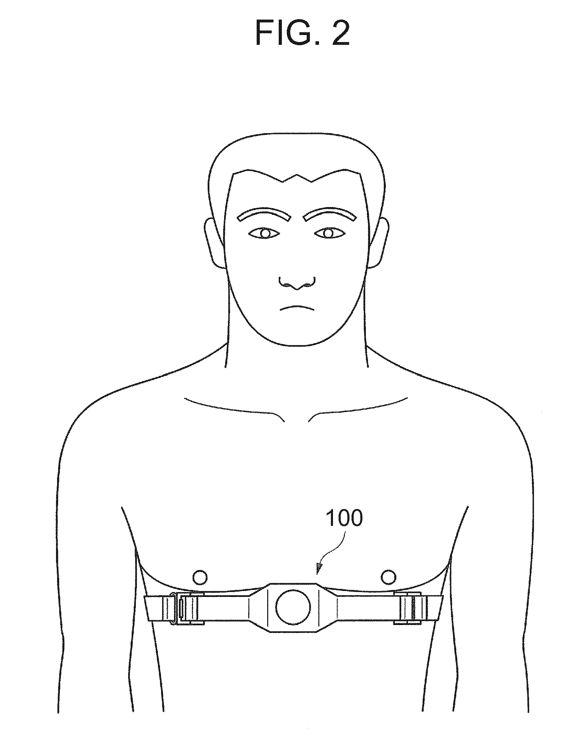

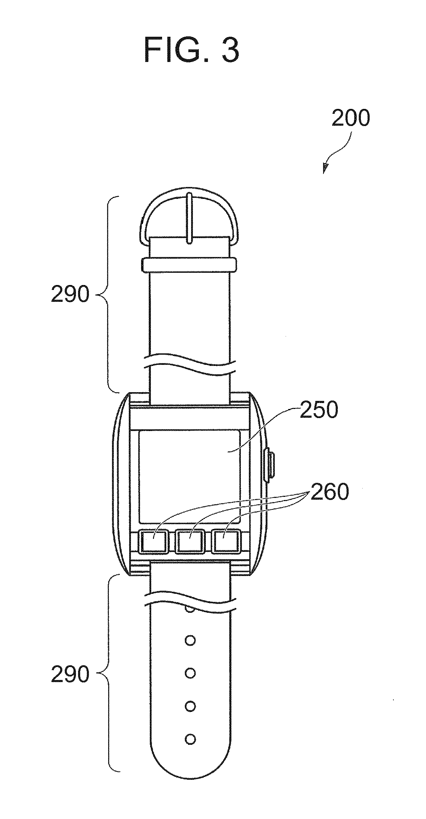

Fig. 1 is a block diagram showing a configuration of a heart rate measurement system 1 according to the embodiment. The heart rate measurement system 1 includes a heart rate measurement device 100 and an output device 200. Fig. 2 is a view showing an example of an outer appearance of the heart rate measurement device 100. As shown in Fig. 2, the heart rate measurement device 100 is formed to have an approximately ring-shape so as to be attached over the entire circumference of a chest portion of a user. The heart rate measurement device 100 is attached to the chest portion of the user by using a band, and a pair or electrodes is allowed to contact the chest portion of a body (the surface of a living body) to thereby detect an electrocardiographic signal generated with heart beats, and the heart rate is calculated based on the detected electrocardiographic signal. Fig. 3 is a view showing an example of an outer appearance of the output device 200. As shown in Fig. 3, the output device 200 is, for example, a wrist watch-type device, which is attached to a wrist (arm) of the user by using a band. The output device 200 is formed to have an approximately ring-shape so as to be attached over the entire circumference of a wrist of the user.

As shown in Fig. 1, the heart rate measurement device 100 is formed by including a power supply unit 110, a measurement unit 120, a transmission circuit 130 and an antenna 140.

The power supply unit 110 supplies electric power to respective units included in the heart rate measurement device 100.

The measurement unit 120 includes an electrode 121, an electrode 122, an attachment detection circuit 123, a heat rate detection circuit 124 and a control unit 125. The measurement unit 120 measures the heart rate of the user at regular intervals and outputs the measured heart rate to the transmission circuit 130.

The electrode 121 and the electrode 122 are a pair of electrodes, which detect the electrocardiographic signal generated by heart beats.

When the user attaches the heart rate measurement device 100 to the chest portion, a skin resistance RH is formed between the electrode 121 and the electrode 122, and electric current corresponding to the skin resistance RH flows between both electrodes.

The attachment detection circuit 123 detects whether the user has attached the heart rate measurement device 100 or not by a voltage based on the electric current flowing between both electrodes, outputting an attachment detection signal indicating the detected result to the control unit 125.

The heat rate detection circuit 124 amplifies the electrocardiographic signal detected by the electrode 121 and the electrode 122, outputting the amplified electrocardiographic signal to the control unit 125.

The control unit 125 detects that the heart rate measurement device 100 is attached to the chest portion of the user based on the attachment detection signal inputted from the attachment detection circuit 123. The control unit 125 calculates the heart rate in accordance with a pulse interval of the electrocardiographic signal outputted from the heat rate detection circuit 124. The pulse interval of the electrocardiographic signal is, for example, an interval between peaks of the electrocardiographic signal.

When it is determined that the heart rate measurement device 100 is attached to the chest portion of the user, the control unit 125 outputs information indicating the calculated heart rate to the transmission circuit 130 as a measurement result. The measurement result is transmitted to the output device 200 through the antenna 140.

The output device 200 is formed by including a power supply unit 210, an antenna (acquisition unit) 220, a receiving circuit (acquisition unit) 230, a control unit 240, a display unit 250, an input unit 260, a notification unit 270, an illumination unit 280 and an attachment unit 290.

The power supply unit 210 supplies electric power to respective units included in the output unit 200 (except the attachment unit 290).

The receiving unit 230 receives information indicating the heat rate transmitted from the heart rate measurement device 100 through the antenna 220, outputting the received information indicating the heart rate to the control unit 240.

The control unit 240 controls respective units included in the output device 200. The control unit 240 also displays the heart rate on the display unit 250 based on the information received from the heart rate measurement device 100. The control unit 240 moves an image associated with the heart rate displayed on the display unit 250 on the display unit 250 as described later.

The display unit 250 is a display which displays information. The display unit 250 is formed by, for example, a liquid crystal display device (LCD). On the display unit 250, for example, time is displayed in addition to the display of the heart rate.

The input unit 260 detects an operation input by the user, outputting information based on the detected result to the control unit 240.

The notification unit 270 is a device of outputting a tone, which is, for example, a speaker. For example, the notification unit 270 may output the tone every time the operation input by the user is detected.

The illumination unit 280 radiates light on the display unit 250 in accordance with the result detected by the input unit 260 so that the user can visually recognize the display unit 250 in a dark place and so on.

The attachment unit 290 is used for attaching the output device 200 to the user's arm, and is, for example, a band.

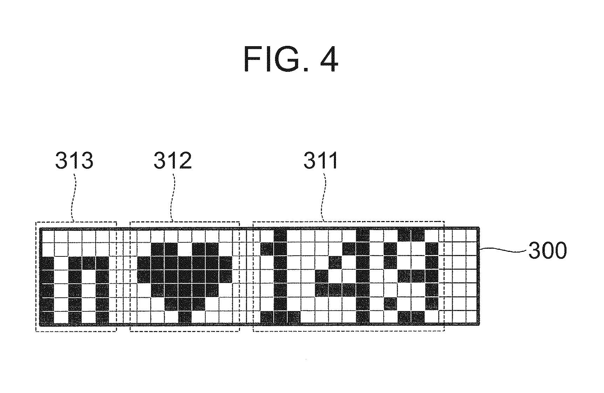

Fig. 4 is a view for explaining an image 300 to be displayed on the display unit 250 according to the embodiment.

As shown in Fig. 4, the image 300 displayed on the display unit 250 includes an image 311 indicating the heart rate, an image 312 associated with the heart rate (hereinafter referred to as a mark) and a character image 313 indicating a state of the heart rate.

The image 311 indicating the heart rate is an image of a value showing the number of beats in one minute.

The mark 312 is displayed on the display unit 250 in a manner in which a display position is moved in a vertical direction in accordance with the heart rate as described later. The mark 312 is an image having an asymmetric shape in the vertical direction with respect to a horizontal line passing through the center of the mark 312, which is, for example, a heart shape. The mark 312 may also have an asymmetric shape as well as a symmetric shape in a horizontal direction with respect to a vertical line passing through the center of the mark 312. The mark 312 may have shapes other than the heart shape.

The character image 313 indicating the state of the heart rate is an image indicating whether the heart rate is within a range between a predetermined lower limit value and an upper limit value of the heart rate, lower than the lower limit value or upper than the upper limit value by character information. For example, an image "IN" is an image displayed when the heart rate is within the range between the predetermined lower limit value and the upper limit value. An image "LO" is an image displayed when the heart rate is lower than the predetermined lower limit value. An image "HI" is an image displayed when the heart rate is higher than the predetermined upper limit value.

The predetermined upper limit value and the lower limit value may be inputted, for example, by the user operating the output device 200. The predetermined upper limit value and the lower limit value may also be determined by being calculated by the control unit 240 based on heart rate information and living body information of the user.

Though only the display relating to the heart rate is shown as an example of the image to be displayed on the display unit 250 in Fig. 4, time and so on may be displayed when the display device 200 has a watch function.

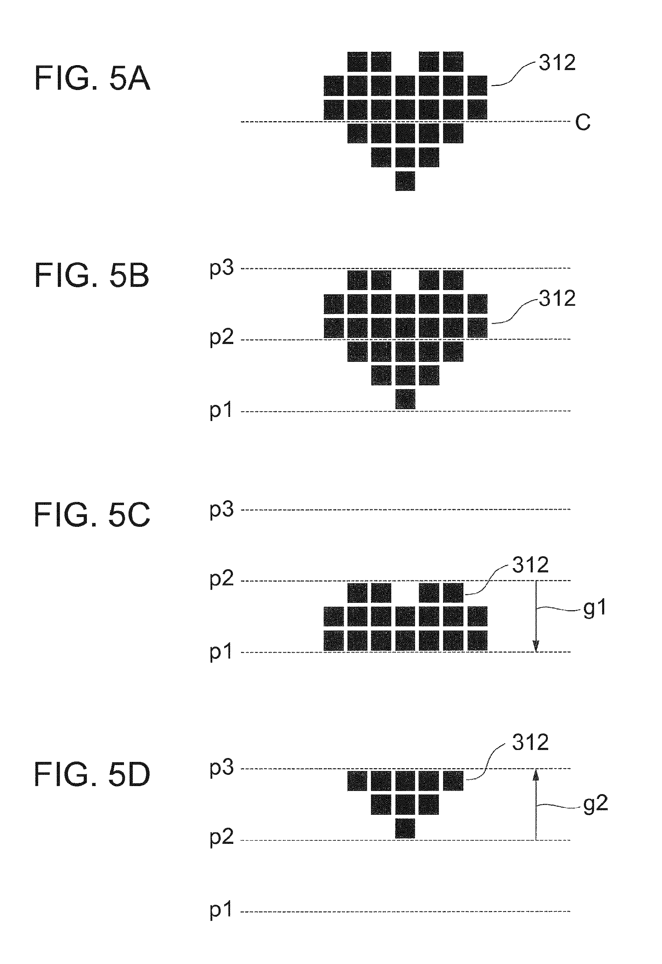

Figs. 5A to 5D are views for explaining the mark 312 according to the embodiment. Fig. 5A is a view for explaining the center position of the mark 312 in the vertical direction, Figs. 5B to 5D are views showing examples in which the mark 312 is displayed at different positions in the vertical direction. In Figs. 5B to 5D, a vertical position p1 is a lower edge position of the image 300, a vertical position p2 is the center of the image 300 in the horizontal direction and a vertical position p3 is an upper edge position of the image 300.

As shown in Fig. 5A, when the number of pixels of the mark 312 in the vertical direction is 6 pixels, the center C (hereinafter referred to as the center position in the vertical direction) of the mark 312 in the vertical direction is a position between the third pixel and the fourth pixel.

The example shown in Fig. 5B shows an image to be displayed when the center position C of the mark 312 in the vertical direction is at the vertical position p2 of the image 300. The vertical position p2 is an intermediate position between the vertical position p3 in the upper edge of the image 300 and the vertical position p1 in the lower edge. The mark 312 will be displayed so that the center thereof overlaps the vertical position p2 as shown in Fig. 5B in the case when the heart rate is in the range between the predetermined lower limit value and the upper limit value of the heart rate.

The example shown in Fig. 5C shows an image to be displayed when the center position C of the mark 312 in the vertical direction is at the vertical position p1 of the image 300. The mark 312 will be displayed so that the center thereof overlaps the vertical position p1 as shown in Fig. 5C in the case when the heart rate is lower than the predetermined lower limit value of the heart rate. In Fig. 5C, the mark 312 is displayed in a state of having been moved downward when one faces the image by a length of an arrow g1 from the state shown in Fig. 5B so that the center position in the vertical direction is moved from the vertical position p2 to the vertical position p1.

The example shown in Fig. 5D shows an image to be displayed when the center position C of the mark 312 in the vertical direction is at the vertical position p3 of the image 300. The mark 312 will be displayed so that the center thereof overlaps the vertical position p3 as shown in Fig. 5D in the case when the heart rate is higher than the predetermined upper limit value of the heart rate. In Fig. 5D, the mark 312 is displayed in a state of having been moved upward when one faces the image by a length of an arrow g2 from the state shown in Fig. 5B so that the center position in the vertical direction is moved from the vertical position p2 to the vertical position p3.

Next, an example of display processing of the mark 312 performed by the control unit 240 of the output device 200 based on the heart rate will be explained.

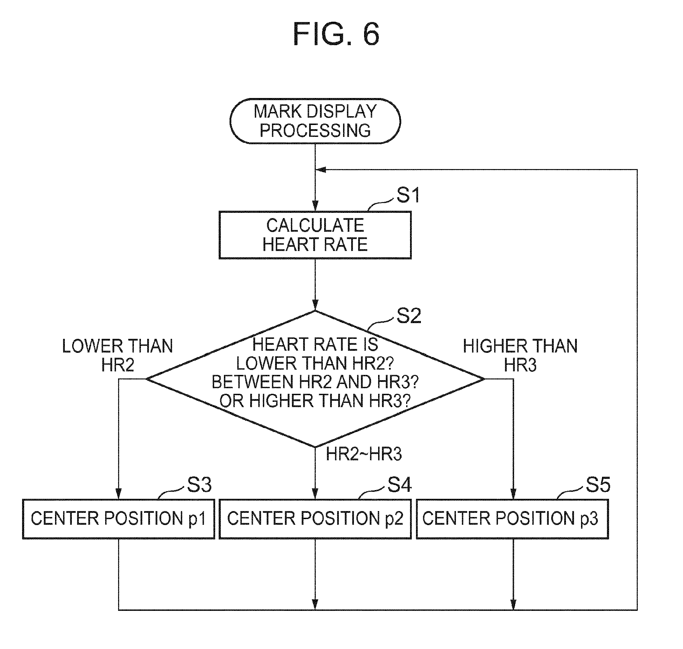

Fig. 6 is a flowchart of a processing procedure of the heart rate measurement system 1 according to the embodiment.
(Step S1) The control unit 125 of the heart rate measurement device 100 calculates the heart rate in accordance with the pulse interval of the electrocardiographic signal outputted from the heat rate detection circuit 124 and transmits information indicating the calculated heart rate to the output device 200.
(Step S2) The control unit 240 determines whether the information indicating the heart rate received from the heart rate measurement device 100 is lower than a predetermined lower limit value HR2 of the heart rate, within a range between the predetermined lower limit value HR2 and an upper limit value HR3, or higher than the predetermined upper limit value HR3. The control unit 240 proceeds to Step S3 when the information indicating the heart rate is determined to be lower than the predetermined lower limit value HR2 of the heart rate (Step S2; lower than HR2). The control unit 240 proceeds to Step S4 when the information indicating the heart rate is determined to be in the range between the predetermined lower limit value HR2 and the upper limit value HR3 of the heart rate (Step S2; HR2 to HR3). The control unit 240 proceeds to Step S5 when the information indicating the heart rate is determined to be higher than the predetermined upper limit value HR3 of the heart rate (Step S2; higher than HR3).
(Step S3) When the information indicating the heart rate is determined to be lower than the predetermined lower limit value HR2 of the heart rate, the control unit 240 moves the mark 312 in the downward direction when one faces the image so that the center position of the mark 312 is positioned at (moves from the vertical position p2 to) the vertical position p1 of the image 300.
(Step S4) When the information is determined to be in the range between the predetermined lower limit value HR2 and the upper limit value HR3 of the heart rate, the control unit 240 positions (does not move) the mark 312 so the center position of the mark 312 is positioned at the vertical position p2 of the image 300.
(Step S5) When the information is determined to be higher than the predetermined upper limit value HR3 of the heart rate, the control unit 240 moves the mark 312 in the upper direction when one faces the image so that the center position of the mark 312 is positioned at (moved from the vertical position p2 to) the vertical position p3 of the image 300.

After that, the heart rate measurement system 1 repeats the processing from Step S1 to S5.

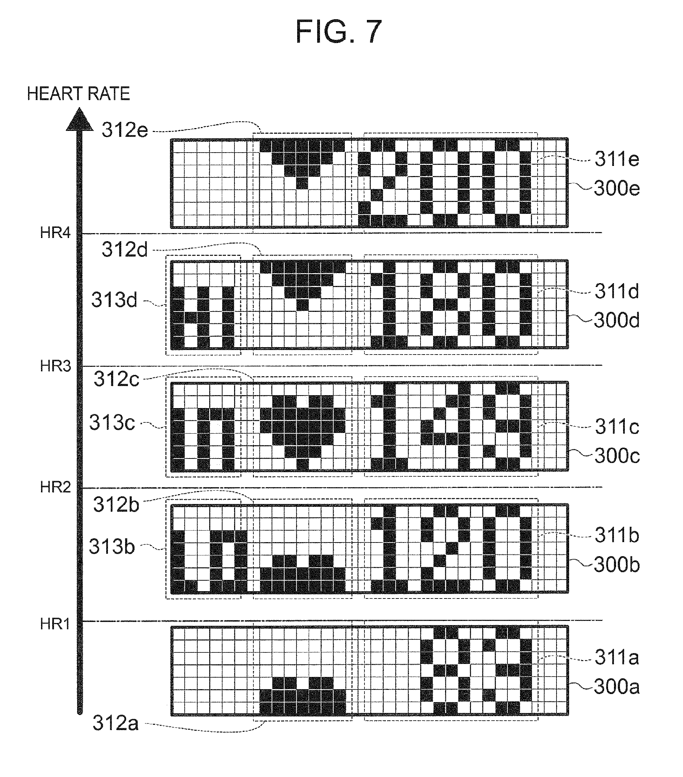

Fig. 7 shows examples of images to be displayed on the display unit 250 in accordance with the heart rate, according to the embodiment.

In Fig. 7, images 311a to 311e are images indicating the heart rate, images 312a to 312e are marks and images 313b to 313d are character images indicating states of the heart rate.

A heart rate value HR1 is a heart rate which is lower than the predetermined lower limit value HR2 of the heart rate and a heart rate value HR4 is a heart rate which is higher than the predetermined upper limit value HR3 of the heart rate. The marks 312a to 312e are generically called the mark 312. The images 300a to 300e are generically called the image 300.

When the heart rate is lower than the value HR1, the mark 312a is displayed in a state of having been moved in the lower direction when one faces the image so that the center position C in the vertical direction is positioned at the vertical position of the lower edge of the image 300a. In this case, the heart rate is lower than the predetermined lower limit value HR2 and is further lower than the predetermined value HR1, therefore, the control unit 125 does not display the character image indicating the state of the heart rate at the left end of the image 300.

When the heart rate is between the value HR1 and the lower limit value HR2, the mark 312b is displayed in state of having been moved in the lower direction when one faces the image so that the center position C in the vertical direction is positioned at the vertical position of the lower edge of the image 300b.

Moreover, a character image "LO" indicating the state of the heart rate is displayed at the left end of the image 300b.

When the heart rate is between the value HR2 and the upper limit value HR3, the mark 312c is displayed without having been moved, so the center position C in the vertical direction is at the vertical position of the center of the image 300c. Moreover, a character image "IN" indicating the state of the heart rate is displayed at the left end of the image 300c.

When the heart rate is between the upper limit value HR3 and the value HR4, the mark 312d is displayed in a state of having been moved in the upper direction when one faces the image so that the center position C in the vertical direction is positioned at the vertical position of the upper edge of the image 300d. Moreover, a character image "HI" indicating the state of the heart rate is displayed at the left end of the image 300d.

When the heart rate is higher than the value HR4, the mark 312e is displayed in a state of having been moved in the upper direction when one faces the image and displayed so that the center position C in the vertical direction is positioned at the vertical position of the upper edge of the image 300e. In this case, as the heart rate is higher than the predetermined value HR4, the control unit 125 does not display the character image indicating the state of the heart rate at the left end of the image 300e. Here, the value HR4 is higher than the upper limit value HR3.

As shown in Fig. 7, when the heart rate is higher than the upper limit value HR3, the mark 312 is displayed on the display unit 250 in the state of having been moved in the upper direction by the control unit 240 so that the mark 312 is seen by the user as if the upper half of the image is cut off and the lower half is adhered to the upper edge of the image 300. When the heart rate is changed from the lower state to the higher state as in this case, the control unit 240 moves the mark 312 in the upper direction and displays the mark 312 on the display unit 250 so that the mark 312 is seen as if the center position thereof is moved from the lower part to the upper part of the image.

As described above, the living body information detection apparatus (heart rate measurement system 1) according to the embodiment includes an acquisition unit (antenna 220, receiving circuit 230) acquiring living body information obtained by detecting a body signal and the control unit 240 moving the display position of the image associated with the living body information on the display unit based on the magnitude of a value of the living body information obtained by the acquisition unit.

According to the above configuration, in the living body information detection apparatus according to the embodiment, the image based on living body information of the user is moved and displayed on the display unit. As a result, the living body information detection apparatus according to the embodiment can allow the information based on the acquired living body information to be easily recognized by the user. Accordingly, the living body information detection apparatus according to the embodiment has an advantage that the user can easily grasp the exercise state of himself/herself intuitively and immediately from the information displayed on the display unit when the user watches the display of the management device while exercising.

There is another advantage that the user can grasp a momentary state intuitively even in a small (narrow) display area by performing display by moving the image as described above.

The example in which the mark 312 is displayed so that the center position of the mark 312 is moved to any of the positions p1, p2 and p3 of the image 300 has been explained in Fig. 5 and Fig. 7, however, the present invention is not limited to this. For example, the control unit 125 can move the central position of the mark 312 at intervals of one pixel or two pixels between the lower edge and the upper edge to perform display. The control unit 240 may also move the mark 312 in right and left directions of the image 300 to perform display. When the mark 312 is moved to right and left to perform display, the control unit 240 may also move the mark 312 also in upper and lower directions. For example, the control unit 240 may display the mark 312 by moving the mark 312 to the left side as well as the lower side inside a display area of the mark 312 in the image 300 when the heart rate is lower than the predetermined lower limit value, and the control unit 240 may display the mark 312 by moving the mark 312 to the right side as well as the upper side inside the display area of the mark 312 in the image 300 when the heart rate is higher than the predetermined upper limit value. In this case, the control unit 240 may also display the mark 312 so that the mark 312 moves in an oblique direction such as from upper right to lower left or from lower left to upper right inside the display area of the image 300. The movement directions in which the mark 312 is moved may be oblique directions from upper left to lower right and vice versa as well as from lower right to the upper left and vice versa.

The control unit 240 may also output a tone from the notification unit 270 in accordance with the position of the mark 312. The control unit 240 may output a first tone having a high frequency from the notification unit 270 in the case of the image 300d (Fig. 7), and may output a second tone having a frequency lower than the first tone from the notification unit 270 in the case of the image 300b (Fig. 7).

The example in which the heart rate measurement device 100 and the output device 200 are separated has been explained in the embodiment, however, it is also preferable that the heart rate measurement device 100 includes the output device 200.

Moreover, the example in which the image 311 indicating the heart rate, the mark 312 and the character image 313 indicating the state of the heart rate are displayed in a state of being arranged in a line in the right and left direction on the display unit 250 as shown in Fig. 4 and Fig. 7 has been explained in the embodiment, however, the present invention is not limited to this. The image 311 indicating the heart rate, the mark 312 and the character image 313 indicating the state of the heart rate may be displayed in a state of being arranged in a vertical direction.

The example in which the heart rate is detected and displayed as the example of the living body information detection apparatus has been explained in the embodiment, however, it is also preferable that a living body signal to be detected may be other living body signals such as brain waves, blood pressure and so on.

The calculation of the heart rate may be performed on the heart rate measurement device 100 side as well as performed on the output device 200 side.

The above output device 200 may be a wrist watch to be attached to the arm of the user. The output device 200 may also be a portable terminal having the display unit such as a cellular phone. In this case, the portable terminal may have the display unit and the control unit, in which the control unit receives information indicating the heart rate from the heart rate measurement device 100, moving the mark 312 and displaying the mark 312 on the display unit based on the received information indicating the heart rate.

The example in which the control unit 240 of the output device 200 sequentially displays the marks 312 on the display unit 250 based on information indicating the heart rate received from the heart rate measurement device 100 has been explained in the embodiment, however, the present invention is not limited to this. For example, it is also preferable that a storage unit is provided in the control unit 240 and the control unit 240 stores information indicating the heart rate obtained when the user performs exercise such as running in the storage unit. Then, the control unit 240 sequentially may read information indicating the heart rate stored in the storage unit and display information such as the mark 312 and the heart rate 311 on the display unit 250 after the exercise by the user.

It is further preferable to perform control by recording a program for realizing functions of the control unit 240 according to the embodiment of the present invention in a computer-readable recording medium, allowing the program recorded in the recording medium to read in a computer system and executing the program, thereby displaying the mark and the like on the display unit 250. The "computer system" in this case includes OS and hardware such as peripheral devices. The computer system" also include a WWW system including an environment of providing (or an environment of displaying) websites. Moreover, the "computer-readable recording media" include transportable media such as a flexible disc, a magneto-optical disc, a ROM and a CD-ROM, and storage devices such as a hard disk included in the computer system. The "computer-readable recording media" also include devices holding the program for a fixed period of time such as a volatile memory (RAM) inside the computer system to be a server or a client when the program is transmitted through networks such as Internet and communication links such as a phone line.

The program can be transmitted from the computer system storing the program in the storage device and so on to another computer system through a transmission medium or transmission waves in the transmission medium. Here, the "transmission media" transmitting the program include media having a function of transmitting information such as networks (communication networks) such as Internet and communication links (communication lines) such as a phone line. The program may be a program for realizing part of the above functions. The programs may also be a so-called difference file which can be realized by combination with a program already storing the above functions in the computing system.

## Claims

1. A living body information detection apparatus (1) comprising:
an acquisition unit (220, 230) for acquiring living body information obtained by detecting a living body signal; and
a control unit (240) for moving a display position of an image (312) associated with the living body information on a display unit (250) based on the magnitude of a value of the living body information obtained by the acquisition unit.

2. The living body information detection apparatus according to claim 1,
wherein the control unit is adapted to move the image associated with the living body information on the display unit in at least one direction of a vertical direction, a horizontal direction and an oblique direction with respect to the display unit.

3. The living body information detection apparatus according to claim 1 or 2,
wherein the control unit is adapted to display part of the image associated with the living body information when the image based on the living body information is moved and displayed on the display unit.

4. The living body information detection apparatus according to any one of claims 1 to 3,
wherein the image (312) associated with the living body information has a shape which is asymmetric in the vertical direction.

5. The living body information detection apparatus according to any one of claims 1 to 4,
wherein the control unit is adapted to output a tone corresponding to a position where the image associated with the living body information is moved on the display unit, from a notification unit (270).

6. The living body information detection apparatus according to any one of claims 1 to 5,
wherein the living body information is information indicating the heartbeat, and
the control unit is adapted to move the display position of the image associated with the heartbeat on the display unit in accordance with the magnitude of the heartbeat.

7. A living body information detection program allowing a computer (240) of a living body information detection apparatus (1) to execute:
a step of moving a display position of an image (312) associated with living body information on a display unit based on the magnitude of a value of the living body information acquired by an acquisition unit (220, 230) which acquires the living body information obtained by detecting a living body signal.
